(19) Europäisches Patentamt
European Patent Office
Office européen des brevets

(11) Publication number : **0 499 376 A1**

# (12) EUROPEAN PATENT APPLICATION

(21) Application number : **92300648.0**

(22) Date of filing : **24.01.92**

(51) Int. Cl.[5] : **C07B 57/00,** C07C 227/30, C07C 229/36, C07C 229/34, C07F 9/30

(30) Priority : **31.01.91 US 649782**

(43) Date of publication of application : **19.08.92 Bulletin 92/34**

(84) Designated Contracting States : **AT BE CH DE DK ES FR GB IT LI NL SE**

(71) Applicant : **HOECHST CELANESE CORPORATION**
**Route 202-206 North**
**Somerville, N.J. 08876 (US)**

(72) Inventor : **Bhattacharya, Apurba**
**301, Cape Aron**
**Corpus Christi, Texas 78412 (US)**
Inventor : **Mott, Graham N.**
**7417 Lake Como**
**Corpus Christi, Texas 78413 (US)**
Inventor : **Durrwachter, John R.**
**757 Wildwood**
**Corpus Christi, Texas 78410 (US)**

(74) Representative : **De Minvielle-Devaux, Ian Benedict Peter et al**
**CARPMAELS & RANSFORD 43, Bloomsbury Square**
**London WC1A 2RA (GB)**

## (54) Precipitation-induced asymmetric transformation of chiral alpha-amino acids and salts thereof.

(57) The present invention provides a method for resolving a mixture of optically active isomers of an α-amino acid to obtain the preferred optically active isomer. The mixture of isomers is contacted with at least one chiral resolving agent in an organic acid, which is at least a partial solvent for the α-amino acid, and in the presence of a mineral acid, which is present in a quantity which does not protonate the $NH_2$ functionality of the α-amino acid to an extent that diastereomer interconversion is substantially prevented, and in the presence of a catalytic amount of an aromatic aldehyde. The contacting results in simultaneous resolution of a desired diastereomer and interconversion of the remaining undesired diastereomer, to provide a very high yield of the desired diastereomer having a very high optical purity.

Fig. 3

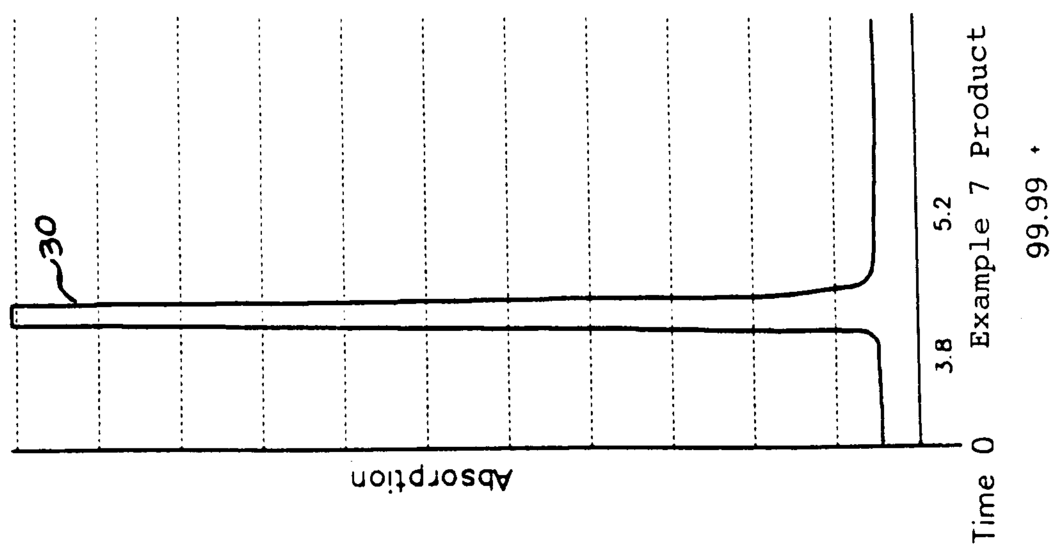


EP 0 499 376 A1

## BACKGROUND OF THE INVENTION

### 1. Field of the Invention

The present invention pertains to a method of resolving a mixture of optically active isomers of an $\alpha$-amino acid to obtain the preferred optically active isomer. In particular, a method is provided by which the optically active D isomer can be obtained from a DL mixture of p-hydroxyphenylglycine.

### 2. Background Art

Although the present invention pertains to a method of resolving an isomeric mixture of an $\alpha$-amino acid, for purposes of example, the method will be described in detail in terms of techniques for resolving the optically active D isomer of DL-p-hydroxyphenylglycine (one of the preferred embodiments of the present invention). Thus, background art related to the resolution of DL-p-hydroxyphenylglycine will also be discussed in detail.

Optically active isomers of p-hydroxyphenylglycine are particularly useful in biochemical/medical applications. For example, D-p-hydroxyphenylglycine is an important starting material in the production of semisynthetic penicillin or semisynthetic cephalosporin. L-p-hydroxyphenylglycine has been shown to be useful in the treatment of ischemic heart diseases, heart failure, and diabetes.

p-Hydroxyphenylglycine does not exist in nature, and is produced exclusively by synthesis. However, the synthesized p-hydroxyphenylglycine produced by methods known to be economically competitive is a DL-isomeric mixture. To obtain a particular optically active isomer from the mixture requires resolution of the DL-isomeric mixture.

The optical purity of a resolved, optically active isomer is important, since this affects the purity of a product produced using the isomer as a starting material. For example, a semisynthetic cephalosporin must meet pharmacological specifications and the optical purity of the D-p-hydroxyphenylglycine used as a starting material for synthesis directly impacts the purity of the product semisynthetic cephalosporin. In addition, the yield of optically active isomer from the resolution process is also important. Methods of resolution which include both optical resolution of the DL-hydroxyphenylglycine and the racemization of the unwanted enantiomer obtained by the optical resolution process provide the best possibility of providing the highest yield of the desired optically active isomer, based on the amount of DL-hydroxyphenylglycine starting material.

J. Tramper et al., in an article entitled "Advantages and Limitations of Chemical Optical Resolution", Biocatalysts in Organic Synthesis, Proceeding of an International Symposium held at Noordwijkerhout, the Netherlands, April 14-17, 1985 and published by Elsevier Science Publishers B.V., Amsterdam, 1985, provides very helpful background art to the present invention. The article describes resolution of a 50:50 mixture of optically active isomers (racemate) using both chemical and enzymatic methods. Within the descriptions of methods of separation by chemical manipulation is the method utilizing of non-covalent diastereomers (diastereomeric salts). This latter method uses a chiral resolving agent which is reacted with the racemate to provide diastereomers whose properties are different, enabling separation via techniques such as crystallization.

Tramper et al. considers the use of non-covalent diastereomers to resolve racemates to be a classical method of resolution. This method provides the advantage that both diastereomers of the racemate can be readily obtained by choice of the appropriate resolving agent for the enantiomer, whereas in enzymatic resolutions, often only one enantiomer is resolved.

Tramper et al. also states that disadvantages of using this classical resolution technique are that it is frequently unpredictable, the processes tend to be rather complex, and impurities can accumulate in the product due to decomposition of the substrate and/or resolving agent during racemization of the undesired diastereomer. "Even relatively small amounts of impurities can have a dramatic effect on the crystallization behavior of the diastereomeric salt." The theoretical, once-through yield (i.e., without racemization of the undesired diastereomer) of a classical resolution is 50%. In practice, chemical yields of greater than 40% of the isomeric mixtures, wherein the product has an optical purity of 95% in a single crystallization are considered good. In general, industrial processes involve racemization of the unwanted enantiomer for economic reasons.

It is possible to obtain once-through yields of greater than 50% (by weight of the isomeric mixture) when the undesired diastereomer remaining in solution undergoes spontaneous equilibration, ie., diastereomer interconversion (the equivalent of racemization of an enantiomer). Such a process is referred to as crystallization induced transformation and has a theoretical yield of 100%. An example of a crystallization induced asymmetric transformation is the resolution of a series of arylglycine esters with tartaric acid in the presence of a carbonyl compound (e.g. benzaldehyde, acetone). The latter catalyzes the racemization of the substrate in solution. Typical yields are in the range of 85-96%, with optical purities of about 95% or greater.

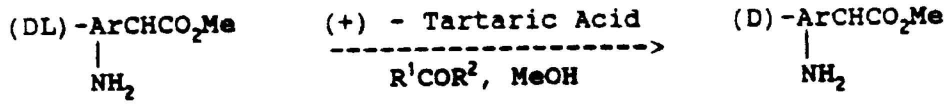


In an article entitled "Method for the Racemization of Optically Active Amino Acid", J. Org. Chem. 48, 843-846, (1983), Yamada et al. describe racemization of a wide variety of optically active α-amino acids, including neutral amino acids, acidic amino acids, basic amino acids, and imino acids by heating in a medium of acetic acid at 80-100°C for 1 hour in the presence of 0.05 molar equivalents of an aliphatic or an aromatic aldehyde. Phenylglycine, (p-hydroxyphenyl)glycine, and serine were racemized without complete dissolution of the optically active isomers.

Yamada et al., Agric. Biol. Chem., 42 (8), 1521-1526 (1978), in an article entitled, "Preparation of D-p-hydroxyphenylglycine: Optical Resolution of DL-p-hydroxyphenylglycine by Preferential Crystallization Procedure", disclose the use of various aromatic sulfonates to form complexes with DL-p-hydroxyphenylglycine, which complexes can be separated using preferential crystallization to provide sulfonates of the desired D-p-hydroxyphenylglycine. Among the various aromatic sulfonates of DL-p-hydroxyphenylglycine, the benzenesulfonate, the o-toluenesulfonate, the p-toluenesulfonate, the p-ethylbenzenesulfonate, the sulfosalicylate, and the 2-naphthol-6-sulfonate are said to be easily resolvable by preferential crystallization. Examples given show yields of the preferred D isomeric complex ranging from about 22% by weight to about 28% by weight (based on the amount of DL sulfonate of p-hydroxyphenylglycine formed in the resolution solution), with optical purity ranging from about 92% to about 97%.

Hongo et al., Bull. Chem. Soc. Jpn., 54, 1905-1910 (1981), in an article entitled, "The Stability of the Supersaturation State in Optical Resolution by the Preferential Crystallization Procedure", describe a preferrential crystallization process for the optical resolution of DL-serine · m-xylene-4-sulfonate. The stability of the supersaturation state of the unseeded isomer was investigated in connection with the cooling conditions. Resolution is achieved using seed crystals of the desired isomer to preferentially crystallize the desired isomer before the spontaneous crystallization of the unseeded, undesired isomer.

Hongo et al., in an article entitled "Racemization of Optically Active Amino Acid Salts and an Approach to Asymmetric Transformation of DL-Amino Acids", Bull. Chem. Soc. Jpn., 3744-3747 (1983), described the racemization process of optically active amino acid salts in connection with optical resolution of DL-amino acids. A wide variety of salts of optically active amino acids with sulfonic acids or mineral acids were racemized by heating in a medium of acetic acid at 80°C to 100°C for one hour in the presence of 0.1 molar equivalents of an aldehyde and free DL-amino acid. From the reaction mixture, the racemized DL-amino acid salts were recovered at a yield of about 86-92 %. An asymmetric transformation between two enantiomers of amino acid salt was attempted by the combination of preferential crystallization of the desired enantiomer and racemization of the antipode in the liquid phase. DL-Alanine · p-chlorobenzenesulfonate was partially converted to L-isomer (yield 16%, optical purity 97%). Another transformation method was carried out by transformation between two diastereoisomeric salts. The combination of selective precipitation of the less soluble diastereoisomeric salt and epimerization of the more soluble diastereoisomeric salt allowed DL-phenylglycine · d-camphor-10-sulfonate to be partially converted to D-p-hydroxyphenylglycine · d-camphor-10-sulfonate (yield 68.1%, optical purity 95.9%).

U.S. Patent No. 4,434,107 to Chibata et al., issued February 28, 1984, discloses the resolution of an optically active p-hydroxyphenylglycine salt with simultaneous racemization of the undesired enantiomer thereof. The invention is carried out by preferential crystallization of the desired enantiomer from a supersaturated solution of the racemic modification thereof, in a lower aliphatic acid in the presence of an aliphatic or aromatic aldehyde. A supersaturated solution of a DL-p-hydroxyphenylglycine salt is prepared in a lower aliphatic acid, the supersaturated solution also contains an aliphatic or aromatic aldehyde. The supersaturated solution is contacted with seed crystals of the desired enantiomer of the p-hydroxyphenylglycine salt at a temperature of not lower than 50°C, thereby allowing preferential crystallization of the desired enantiomer to take place from the supersaturated solution, with simultaneous racemization of the other enantiomer dissolved therein. The salt can be converted into its free form by treating it with an alkali agent or an ion-exchange resin. Despite representations that the method described makes it possible to convert all the modified racemate into a desired enantiomer of the p-hydroxyphenylglycine salt (because the optical resolution of a DL-p-hydroxyphenylglycine salt takes place simultaneously with the racemization of the undesired enantiomer dissolved in liquid media, and at the same time the crystals of the desired enantiomer once preferentially crystallized do not racemize under the physical conditions under which the method is carried out), the Examples show that the yield of the desired enantiomer ranges from about 8% to about 46% by weight, based on the amount of p-hydroxyphenylglycine salt starting material, and the optical purity ranges only from 96.2% to 98%. This optical purity is not adequate

to use the D-p-hydroxyphenylglycine (DHPG) produced by this method as a starting material in the synthesis of amoxycilin. Amoxycilin synthesis methods require a DHPG optical purity of at least 99.1% due to pharmacological requirements and limitations.

Hongo et al., in a paper entitled "Asymmetric Transformation of DL-p-Hydroxyphenylglycine by a Combination of Preferential Crystallization and Simultaneous Racemization of the o-Toluenesulfonate", Bull. Chem. Soc.,Jpn., 58, 433-436 (1985) disclose the assymetric transformation of DL-p-hydroxyphenylglycine between two enantiomers by a combination of preferential crystallization of a desired enantiomer of p-hydroxyphenylglycine · o-toluenesulfonate (HPG · o-TS) and the simultaneous racemization of the antipode. Hongo et al. claim the L-HPG · o-TS was easily racemized by heating at 100°C in aqueous 95% (v/v) acetic acid in the presence of small amounts of salicylaldehyde and free DL-HPG. A supersaturated solution of DL-HPG · o-TS placed under such conditions for racemization was seeded with the crystals of p-HPG · o-TS, and added with DL-HPG and o-toluenesulfonic acid in order to provide continuously the supersaturated state of DL-HPG · o-TS as a driving force. A yield of 77.2% D-isomer, based on the DL-HPG added was obtained.

There is a basic problem in the use of crystallization as the technique for removing the desired enantiomeric salt from its solvent. As the undesired enantiomeric salt becomes more concentrated in the solvent, the risk of crystallizing the undesired enantiomeric salt along with the desired enantiomeric salt increases, lowering both the yield and the optical purity of the desired enantiomeric salt.

Yamada et al., Agria. Biol. Chem. 43 (2) 395-396 (1979), in an article entitled "Preparation of D-p-Hydroxyphenylglycine · Optical Resolution of DL-p-hydroxyphenylglycine with d-3-bromocamphorsulfonic Acid", describe the optical resolution of D-p-hydroxyphenylglycine (D-HPG) using the classical non-covalent diastereomers resolution technique described previously herein, with d-3-bromocamphorsulfonic acid (D-BCA) being the resolving agent. After the D-HPG was crystallized and filtered from the resolving solvent, the remaining salt which was produced by reaction of the L isomer of p-hydroxyphenylglycine with the d-3-bromocamphorsulfonic acid, L-HPG-d-BCS, was hydrolyzed to L-HPG. The L-HPG was subsequently racemized (autoclave, 140°C) and recycled as starting DL isomeric mixture. The yield of D-p-hydroxyphenylglycine (D-HPG) was about 41% based on the amount DL-HPG starting material. Optical purity of the D-HPG was about 99.99%.

U.S. Patent No. 4,415,504 to Chibata et al., issued November 15, 1983, discloses the formation of p-hydroxyphenylglycine-α-phenylethanesulfonate which is useful for the production of optically active p-hydroxyphenylglycine and for the production of optically active α-phenylethanesulfonic acid. The p-hydroxyphenylglycine-α-phenylethanesulfonate was produced by either reacting DL-p-hydroxyphenylglycine with optically active α-phenylethanesulfonic acid, or by reacting (±)-α-Phenylethanesulfonic acid with optically active p-hydroxyphenylglycine. Two diastereomers of DL-p-hydroxyphenylglycine optically active α-phenylethanesulfonate are formed or optically active p-hydroxyphenylglycine (±)-α-phenylethanesulfonate is formed, and one diastereomer is isolated according to the difference between solubilities of the diastereomers. In the claimed method, DL-p-hydroxyphenylglycine is reacted with optically active α-phenylethanesulfonic acid to form two diastereomers of the optically active p-hydroxyphenylglycine · optically active α-phenylethanesulfonate, one of which is easily soluble in the aqueous reaction medium and the other of which is only slightly soluble. The slightly soluble diastereomer is crystallized and the crystals are collected (filtered off and dried). When the crystals comprised D-p-hydroxyphenylglycine (+) · α-phenylethanesulfonate, the crystals were subsequently combined with methanol, and to this mixture was added an aqueous sodium hydroxide solution, whereby the pH of the mixture was adjusted to 6. The mixture was stirred at room temperature for about 2 hours and then filtered to provide D-p-hydroxyphenylglycine (D-HPG). Although the optical purity of the D-HPG produced was 99.8%, the yield of D-HPG, calculated on the basis of the DL-p-hydroxyphenylglycine (DL-HPG), ranged only from about 57-80%.

Yoshioka et al., in a paper entitled "The Optical Resolution and Asymmeric Transformation of DL-p-Hydroxyphenylglycine with (+)-1-Phenylethanesulfonic Acid", Bull. Chem. Soc. Jpn., 60, 649-652 (1987), describe a method for optically resolving DL-HPG to D-HPG by fractional crystallization of a diastereomeric salt with (+)-1-phenylethanesulfonic acid, wherein soluble L-HPG · (+)-phenylethanesulfonate (PES) was easily epimerized into DL-HPG · (+)-PES by heating it at 100°C in glacial acetic acid in the presence of a small amount of salicylaldehyde. Under such epimerizing conditions, the asymmetric transformation of DL-HPG · (+)-PES was attempted with simultaneous fractional crystallization of the less soluble D-HPG · (+)-PES and epimerization of the soluble L-HPG · (+)-PES. A maximum yield of about 80% D-HPG, based on the DL-HPG starting material was achieved; the optical purity of the D-HPG was 95.0%; and the rate of epimerization of the L-HPG · (±)-PES was such, under the method conditions, that about 4 hours time was required for 100% epimerization to DL-HPG · (+)-PES. One principal problem here is that the (+)-PES is not commercially available, and preparation of (+)-PES from (±)-PES required the use of D-HPG as a resolving agent. This defeats the purpose of the process, since the desired product must be used to prepare a starting material which is used on a 1:1 basis to produce

the desired product.

U.S. Patent No. 4,647,692 to Jacewitz, issued March 3, 1987 discloses a process for racemization of amino acids by use of a ketone and an organic acid such as acetic acid. In particular, a process for the racemization of an optically active α-amino acid is described wherein the α-amino acid is treated with a ketone in the presence of a $C_{1-6}$ alkanoic acid. The process is extended to the preparation of an optically active α-amino acid, which comprises treating a racemic α-amino acid with 3-bromocamphor-9-sulphonic acid in the presence of a ketone and a $C_{1-6}$ alkanoic acid. The latter process comprises permitting a 3-bromocamphor-9-sulphonate salt of an optically active 4-hydroxyphenylglycine or 3,4 dihydroxyphenylglycine to crystallize from a solution of a mixture of diastereoisomeric camphorsulphonate salts, in the presence of a ketone and an organic acid; and liberating the optically active 4-hydroxyphenylglycine or 3,4-dihydroxyphenylglycine. In this way, preferential crystallization of one of the diastereomers takes place and the remaining salt is racemized in situ. The ketone may be employed in excess, i.e. 0.5 to 20 mole, preferably 1 to 10 mole per mole of α-amino acid. The $C_{1-6}$ alkanoic acid is used at a concentration from 5 to 50 moles of organic acid per mole of α-amino acid. The organic acid, together with the ketone, may conveniently form the solvent for the racemization process. In the single Example, 8, which demonstrates optical resolution being carried out simultaneously with racemization to produce a desired diastereomer, it was necessary to conduct several independent resolutions of the starting racemic 3,4-dihydroxyphenylglycine monohydrate, with each resolution step being followed by a crystallization step and removal of the crystallized bromocamphor-9-sulphonate salt of the glycine. The salt produced was about a 42% yield, based on the racemic 3,4-dihydroxyphenylglycine starting material. The combined filtrate and washings from the independent optical resolution steps was then racemized and further resolved in the presence of toluene and sodium acetate in a two step process over a time period of about 19 hours, to provide a total yield of salt for the entire process of about 83.7%, based on the racemic 3,4-dihydroxyphenylglycine starting material.

Yoshioka et al., Chem. Pharm. Bull. 37(4) 883-886 (1989) in an article entitled "Efficient preparation of D-Aspartic Acid β-Methyl Ester as an Aspoxicillin Material by Optical Resolution, Epimerization and Asymmetric Transformation", disclose a second-order asymmetric transformation. The diastereomeric resolution of DL-Asp (OMe) with (-)-1-phenylethane-sulfonic acid (PFS) resulted in salt formation of less soluble D-·(-) and more soluble L-·(-) in acetonitrile. The soluble L-·(-) was easily epimerized into DL-·(-) by heating it in acetonitrile in the presence of catalysts. Attempted fractional crystallization of DL-·(-) or L-·(-) under such epimerizing conditions made possible a 90% yield of D-·(-) via equilibrium asymmetric transformation in a solid-liquid heterogeneous system. This yield is affected in part by the high solubility of the diasteriomer in the chosen solvent system.

Shiraiwa et al., in an article entitled "Asymmetric Transformation of N-Methyl-(RS)-2-phenylglycine", The Chemical Society of Japan, Chemistry Letters, 233-234 (1990) describes the asymmetric transformation of N-methyl-(RS)-2-phenyl glycine ((RS)-Mpg) via formation of the salt with (S)-camphor-10-sulfonic acid in butanoic acid without using a catalyst such as aldehydes or ketones. The salt, purified by recrystallization, was treated with triethylamine to give (R)-Mpg at a yield of 71% - 77%, the salt having an optical purity ranging from 98.4% to 99.0%. Higher yields, up to 91.9% were obtained at lower optical purities ranging from about 55.9% up to 87.9%.

In view of the above background art, it is apparent that it would be highly desirable to have a "single pot" process wherein optical resolution of a mixture of α-amino acid isomers is carried out via simultaneous asymmetric transformation of at least one isomer to a desired diastereomer salt with diastereomer interconversion of the undesired diastereomer salt, whereby all of the α-amino acid is converted to the desired diastereomer salt. The salt can then be converted to the desired enantiomer. The optical purity of the enantiomer produced must be adequate to meet the intended end use requirements. The direct yield, based on the starting isomeric mixture of α-amino acid, must be economically satisfactory or the process streams must be capable of being recycled in a manner to generate a satisfactory effective yield. The time period required to achieve the satisfactory yield must be such that the process is economically competitive.

## BRIEF DESCRIPTION OF THE DRAWINGS

Figures 1-3 show high pressure liquid chromatograms (HPLC) of isomeric mixtures of DL-p-hydroxyphenylglycine (DL-HPG).

Figure 1 shows an HPLC for a 50:50 racemic mixture of DL-HPG.

Figure 2 shows an HPLC for a commercially available D-p-hydroxyphenylglycine (D-HPG) which is available from Aldrich Chemical Co., Inc. This product comprises about 99.5% by weight of D-HPG and about 0.5% by weight L-p-hydroxyphenylglycine (L-HPG).

Figure 3 shows an HPG for D-HPG prepared using the method of the present invention, as described in EXAMPLE 7. This D-HPG exhibited an optical purity of 99.99+ % by weight.

## SUMMARY OF THE INVENTION

In accordance with the present invention, a method is provided for resolving a mixture of optically active isomers (typically a racemate) of an α-amino acid (or of a salt thereof) to obtain the preferred optically active isomer. The method makes it possible to obtain a particular optically active isomer from a mixture of isomers at a yield in excess of 99% by weight of the mixture, in a reasonable time period (less than about 24 hours). Typically, a 99% yield can be obtained in about 6 hours, and in some cases in as little as 2 hours. The method preferably combines precipitation-induced asymmetric transformation with simultaneous interconversion of the undesired isomer, making possible the high yield based on the amount of α-amino acid isomeric mixture.

The α-amino acid isomeric mixture, typically a 50:50 racemic mixture, is contacted with a chiral resolving agent in an organic acid which is at least a partial solvent for the α-amino acid mixture, in the presence of a mineral acid, and in the presence of a catalytic amount of an aromatic aldehyde.

The chiral resolving agent reacts with the racemic mixture of the α-amino acid to form two diastereomeric salts, one which is the desired diastereomeric salt and the other which is the undesired diastereometric salt. The undesired diastereomeric salt must be converted back to a form of the racemic α-amino acid mixture which can be further converted into the desired diastereometric salt. This conversion process is subsequently referred to herein as diastoreomer interconversion.

The chiral resolving agent selected depends on the particular α-amino acid isomeric mixture to be resolved. Typically, chiral sulfonic acids and/or chiral carbonic acids work well. (+)-3-Bromocamphor-8-sulfonic acid, ammonium salt (BCSA) is a particularly good chiral resolving agent for DL-p-hydroxy-phenylglycine (HPG). Examples of chiral resolving agents used in the resolution of amino acids include, for example: the resolution of DL-Lysine using an acid selected from the following: (+)-camphoric, (+)-camphor-10-sulfonic, (+)-α-bromocamphor-π-sulfonic, (-)-6,6′-dinitrodiphenic, and N-acetyl, 3,5,-dibromo-L-tyrosine and D-tyrosine; the resolution of DL-methionine using (+)-α-bromocamphore-π-sulfonic acid; the resolution of DL-histidine using d-tartaric acid; the resolution of DL-phenylglycine using optically active β-hydroxysulfonates such as (-)-sodium (1S, 2S, 4R)-2-hydroxy-p-menthane-1-sulfonate; the resolution of α-aminophenylglycine using D-camphorsulfonic acid; and the resolution of α-amino-3-thienylacetic acid using α-bromo-D-camphor-π-sulfonic acid and D-camphor-10-sulfonic acid. The literature contains numerous examples of and data pertaining to chiral resolving agents for particular α-amino acids.

The organic acid used as the reaction medium for the resolution-racemization can be selected from the group consisting of $C_1$ to $C_6$ alcanoic acids, and preferably from aliphatic organic acids having from 2 to 6 carbon atoms, and most preferably from aliphatic organic acids having from 2 to 4 carbon atoms.

A strong mineral acid is used to protonate the $NH_2$ functionality of the α-amino acid, enabling the formation of the diastereometric salt. Examples, not intended to be limiting, of the strong mineral acid, include hydrochloric acid, sulfuric acid, phosphoric acid, trifluoromethane sulfonic acid, and toluene sulfonic acid; preferred are hydrochloric acid, sulfuric acid, or phosphoric acid.

The rate at which the strong mineral acid is added must be carefully controlled, because the diastereomer interconversion cannot take place if all of the $NH_2$ functionality of the α-amino acid becomes protonated. In fact, the rate at which the diastereomer interconversion can take place is a function of the amount of remaining, unprotonated $NH_2$ functionality of the α-amino acid.

Alternatively, the strong mineral acid can be replaced by a weaker acid which does not protonate the $NH_2$ function of the amino acid to such a high degree. When the weaker acid is used, the acid addition rate is not critical. The disadvantage of using the weaker acid is that the diastereometric salt forms more slowly. Examples of weaker acids which can be used include, but are not limited to, trifluoroacetic acid and trichloroacetic acid.

Examples of aromatic aldehydes which can be used in a catalytic amount include, but are not limited to salicylaldehyde, 3,5-dichlorobenzaldehyde, and m-nitrobenzaldehyde. Most preferably, salicyaldehyde is used. The resolution-racemization process proceeds more rapidly if the aromatic aldehyde comprises an electron-withdrawing group on the aromatic ring.

The product formed by the method of the present invention is a diastereomer salt of the amino acid which can be converted to the desired amino acid isomer by hydrolysis. The hydrolysis can be carried out using either an organic base or an inorganic base in an aqueous or alcoholic solvent. The precipitated diastereomer salt forms a slurry in the aqueous or alcoholic solvent where it is contacted with the hydrolyzing agent.

The optical purity of the desired diastereometric salt obtained using the method of the present invention is improved when water is not present during the resolution-diastereometric interconversion process. Water acts as a solvent for the DL isomeric mixture, leaving the mixture dissolved in water as an impurity which does not take part in the resolution-diastereometric interconversion process. As the desired diastereomeric salt is precipitated from the organic acid solvent, this impurity can be carried out with the desired diastereomeric salt, ultimately lowering the optical purity of the desired isomer product. Any water present in the organic acid used

as solvent can be removed by adding an anhydride to the resolution-interconversion mixture. An example of the anhydride is the anhydride of the organic acid solvent. The amount of anhydride used is typically less than 5% by weight of the organic acid solvent. The organic acid solvent typically contains about 1% by weight water or less and the amount of anhydride needs be only present in one equivalent based on the amount of water.

The effect of water on the optical purity of the desired isomer product was demonstrated for DL-p-hydroxyphenylglycine (DL-HPG) as follows. When the resolution-interconversion mixture (DL-HGP, acetic acid, sulfuric acid, salicylaldehyde and (+)-3-bromocamphor-8-sulfonic acid) contained 0.1% by weight water, the optical purity of the obtained D-HPG obtained was about 88%; when the water content was 0.01% by weight, the optical purity of the D-HPG obtained was about 98%; when the water content was 0.001% by weight, the optical purity of the D-HPG obtained was about 99%.

The method of the present invention then comprises the steps of:

a) contacting an optically active isomeric mixture of an α-amino acid or a salt thereof with at least one chiral resolving agent, in an organic acid which is at least a partial solvent for the α-amino acid and in the presence of a mineral acid which is present in a quantity which does not protonate the $NH_2$ functionality of the α-amino acid to an extent that diastereomer interconversion is substantially prevented, and in the presence of a catalytic amount of an aromatic aldehyde; and,

b) simultaneously carrying out resolution of a desired diastereomer formed by the reaction of the α-amino acid with the chiral resolving agent while obtaining diastereomer interconversion of the undesired diastereomer, wherein said resolution-interconversion is carried out at a sufficient temperature to provide an economically satisfactory resolution time period.

The yield of the desired diastereomer, based on the quantity of racemic mixture, depends upon the rate/amount of addition of mineral acid and upon the time period over which the optical resolution-interconversion is carried out. Typically, when one equivalent of strong mineral acid, based on the amount of isomeric mixture, is added over the resolution-interconversion processing period, and the reaction temperature is approximately the boiling temperature of the organic acid solvent (typically about 60°C to 120°C), the time period required to obtain at least a 99% by weight yield of the desired diastereomer ranges from about 2 hours to about 24 hours. The reaction is typically slightly endothermic and once the reaction mixture is heated to the desired reaction temperature, only a small amount of heat is needed to maintain this temperature.

The diastereomer can then be converted to the desired isomer by hydrolysis, as previously described.

## DETAILED DESCRIPTION OF THE INVENTION

The method of the present invention pertains to the resolution of optically active isomeric mixtures of α-amino acids.

The α-amino acid (or salt thereof) is contacted with a chiral resolving agent in the presence of an organic acid which is at least a partial solvent for the α-amino acid, in the presence of a mineral acid, and in the presence of a catalytic amount of an aromatic aldehyde.

The α-amino acid reacts with the chiral resolving agent in the organic acid reaction medium to form two diastereomer salts. One of the diastereomers is the salt of the desired isomer of the α-amino acid. The other, undesired diastereomer must be interconverted to the desired diastereomer.

In the present process both the resolution of the diastereomers and the interconversion of the undesired diastereomer are carried out simultaneously under conditions which permit the desired diastereomer salt to precipitate from the organic acid solvent. Use of precipitation of the desired diastereomer salt during formation of the mixture of diastereomer salts, rather crystallization of the desired diaestereomer salt from solution upon completion of formation of all of the desired diaestereomer salt, helps shift the equilibrium of the interconversion step, leading to higher yields of the desired diastereomer in a shorter time period.

The resolution-interconversion process of the present invention is carried out by adding a mineral acid, typically a concentrated mineral acid, directly to a medium comprising the α-amino acid, chiral resolving agent, organic acid solvent, and a catalytic amount of aromatic aldehyde. The amount of mineral acid added, relative to the amount of α-amino acid $NH_2$ functionality remaining at any time during the resoluting-interconversion, is critical. For purposes of illustration, and not by way of limitation, this criticality will be illustrated for the resolution of DL-p-hydroxyphenylglycine (DL-HPG), wherein the mineral acid is concentrated sulfuric acid, the chiral resolving agent is D-ammonium bromocamphor sulfonate (D-ABCS), the organic acid is acetic acid, and the aromatic aldehyde is salicylaldehyde.

The desired product formed by the resolution-interconversion is a diastereomeric salt, D-HPG · D-BCS. This diastereomeric salt can subsequently be converted to D-HPG by hydrolysis.

The resolution-interconversion is represented as follows:

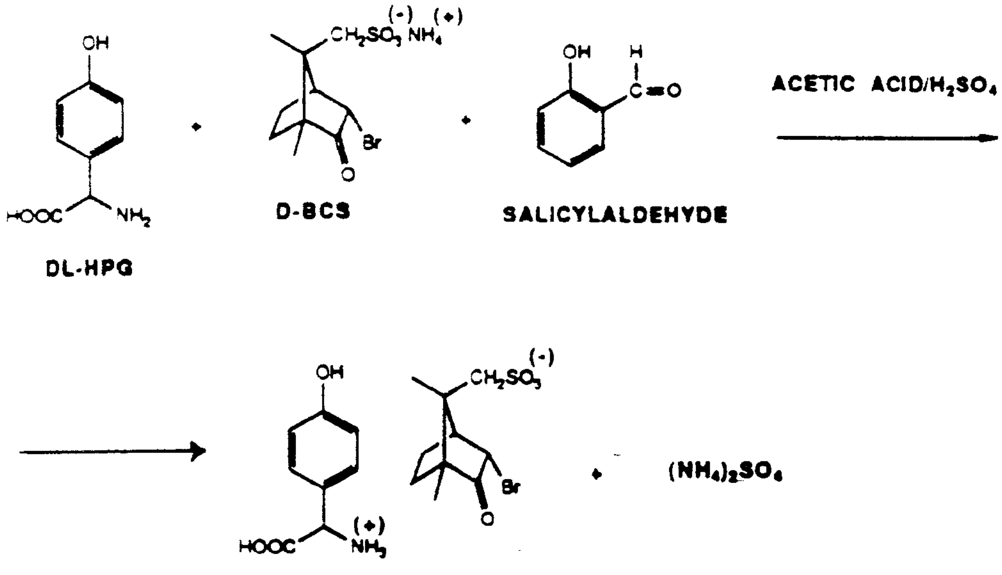


DL-HPG

D-BCS

SALICYLALDEHYDE

D-HPG.D-BCS

The process involves simultaneous precipitation of the D-HPG·D-BCS and diastereomer interconversion of L-HPG·D-BCS to DL-HPG. It is critical to the economic feasibility of the process that the interconversion step be rapid.

It is believed the interconversion mechanism is as follows. The L-HPG portion of the L-HPG·D-BCS salt is converted to L-HPG, which is then converted, in the presence of salicylaldehyde, to an imine and to DL-HPG.

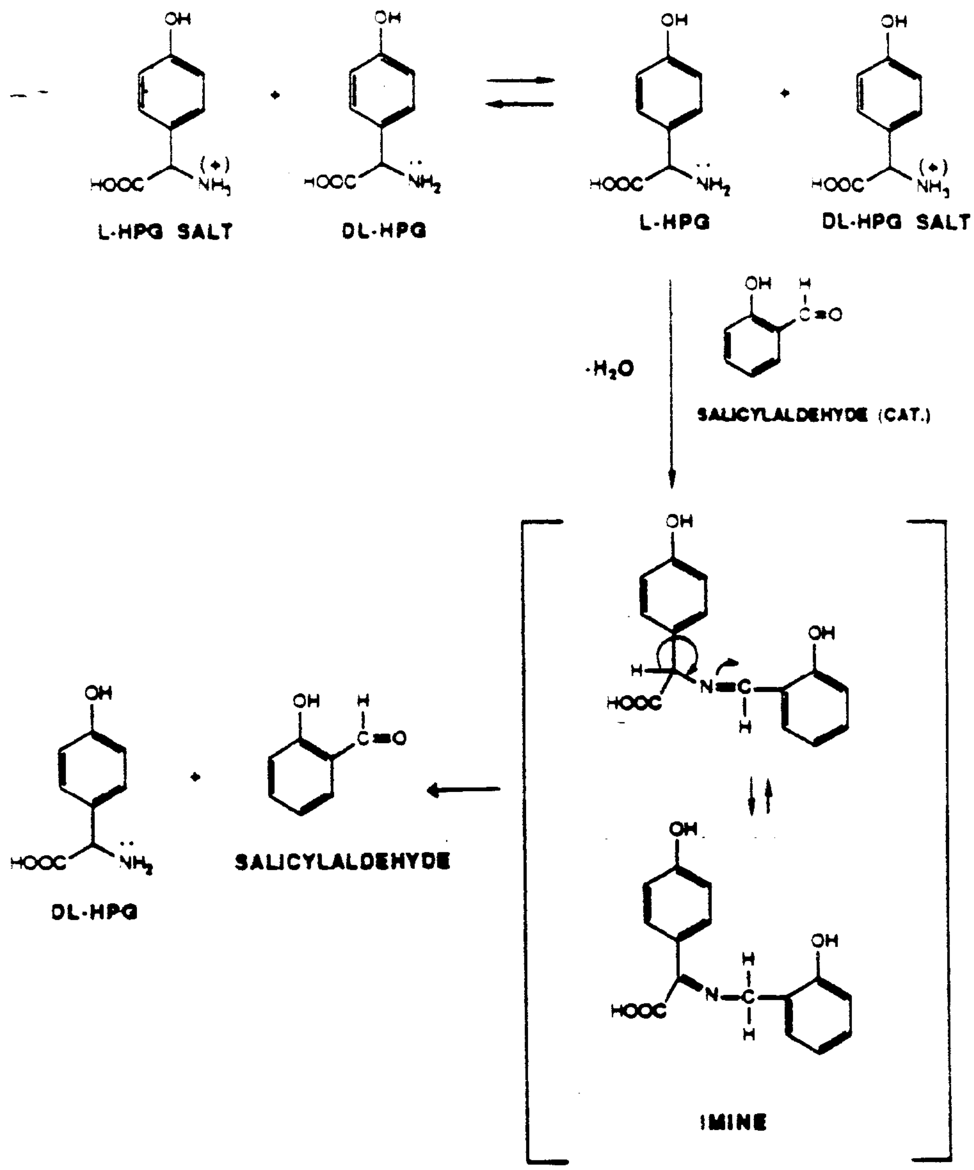


The DL-HPG can be reacted with the D-ABCS or D-BCS to produce more D-HPG·D-BCS. The availability of the lone pair of electrons on the unreacted, unprotonated DL-HPG is crucial for the imine formation which must take place to convert L-HPG to DL-HPG. Applicants discovered that addition of an amount of $H_2SO_4$ which protonates the $NH_2$ functionality of the $\alpha$-amino acid to an extent that diastereomer interconversion is substantially prevented, reduces the yield of D-HPG, and simultaneously affects the optical purity of the D-HPG which is obtained. Addition of one equivalent or more of sulfuric acid at the beginning of the resolution-interconversion process substantially prevents diastereomer interconversion.

When less than one equivalent of sulfuric acid is added at the beginning of the resolution-interconversion, this provides a window through which interconversion is accomplished. Experiments have demonstrated that an initial addition of 0.95 equivalent of sulfuric acid provides a useful window for interconversion, when the process is conducted at atmospheric pressure at a temperature ranging from about 60°C to about 120°C. The remaining sulfuric acid can be added as the process progresses.

Table 1 summarizes the results of several resolution-interconversion experiments which were carried out in acetic acid at 80°C. The amount of acetic acid used was 4 volumes per volume of DL-HPG.

TABLE 1

RESOLUTION-INTERCONVERSION OF DL-HPG[1] TO PRODUCE D-HPG[2] AS A FUNCTION OF THE AMOUNT OF MINERAL ACID ADDED TO THE RESOLUTION MEDIUM

| Example No. | Amount of $H_2SO_4$ (equivalents)[3] | Amount of DL-HPG[1] (mol) | Amount of D-ABCS[4] (mol) | Diastereomeric Excess[5] (%) | Conversion[6] To D-HPG (% by wt) |
|---|---|---|---|---|---|
| 1 | 0.4 | 1 | 1 | 40 | 40 |
| 2 | 0.6 | 1 | 1 | 60 | 60 |
| 3 | 0.8 | 1 | 1 | 80 | 80 |
| 4 | 2.0 | 1 | 1 | 20 | 60 |
| 5 | 4.0 | 1 | 1 | 5 | 52.5 |
| 6 | 20.0 | 1 | 1 | 0 | 50.0 |

[1] racemic mixture of DL-p-hydroxyphenylglycine

[2] isomer D-p-hydroxyphenylglycine

[3] equivalents based on the amount of DL-HPG initially present

[4] D-ammonium bromocamphorsulfonate

[5]
$$\frac{[D\text{-}HPG \cdot D\text{-}BCS] - [L\text{-}HPG \cdot D\text{-}BCS]}{[D\text{-}HPG \cdot D\text{-}BCS] + [L\text{-}HPG \cdot D\text{-}BCS] + [DL\text{-}HPG]} \times 100$$

[6]
$$\frac{[D\text{-}HPG \cdot D\text{-}BCS]}{[D\text{-}HPG \cdot D\text{-}BCS] + [L\text{-}HPG \cdot D\text{-}BCS] + [DL\text{-}HPG]} \times 100$$

The diastereomeric excess was measured by quenching a portion of the resolution-interconversion mixture and analyzing the quenched mixture using a chiral Crownpak$^R$ (CRT) column obtained from J. T. Baker, Inc.

All diastereomeric excess and conversion measurements shown in Table 1 were made after 15 minutes of reaction time. The resolution-interconversion was carried out at 80°C, under atmospheric pressure. Further reaction of the Examples 4 and 5 mixtures for a time period of about 2 to 3 weeks provided a 90% diastereometric excess (conversion of 95% by weight).

This shows that the process becomes uneconomical unless a sufficient interconversion window is left open.

Example 6

An alternative to sequential addition of a strong mineral acid is the use of a weak mineral acid, as previously discussed. One and one-half (1½) equivalents of trifluoroacetic acid (used in place of sulfuric acid), added in total at the beginning of the process as described above, provided a conversion of greater than 99% by weight in a time period ranging from about 10 to about 18 hours.

EXAMPLE 7

Precipitation-induced Asymmetric Transformation: Stereo-specific Synthesis of D-p-Hudroxyphenylglycine (D-HGP)

A mixture of DL-HPG (400 g., 2.39 mol) and acetic acid (3,832 ml) was placed in a 12 liter, 3-neck round bottom flask equipped with a mechanical stirrer, a thermowell, a heating mantel, and a pressure equilization dropping funnel capped with a nitrogen inlet and bubbler system. The flask and auxiliary vessels were under

a nitrogen blanket at atmospheric pressure.

To the DL-HPG/acetic acid mixture was added 814 g (2.59 mol) of (+)-3-bromo-9-camphor-sulfonic acid ammonium salt (D-ABCS); subsequently, 1,076 ml of 1 M sulfuric acid in acetic acid was added, followed by 8.8 ml of salicylaldehyde, to form a reaction slurry.

The above described mixture was stirred and maintained at a temperature of 70°C. The reaction between the DL-HPG and the D-ABCS was carried out for approximately a 30-minute period.

One hundred and seventy (170) ml of a 1 M sulfuric acid in acetic acid solution was placed in the dropping funnel and this solution was added to the reaction mixture in the round-bottomed flask over a two hour time period. At the end of the two-hour time period 32.7 g. of acetic anhydride was added to the flask. The slurry in the flask was then maintained under agitation at about 70°C for an additional 18-hour time period, after which the contents of the flask were cooled to 22°C.

The D-HPG·D-BCS was collected from the slurry by filtration. The D-HPG·D-BCS filtrant was washed using about 1,200 ml of chilled glacial acetic acid. (Optionally, the D-HPG·D-BCS was dried at 60°C under a vacuum of about 20 inches of mercury for a time period of about 15 hours.) 1,133 g. (99% theoretical yield) of D-HPG·D-BCS was obtained, having a diasterioisomeric purity of 99.9% by weight. The diastereomeric purity was measured by a chiral HPLC method using a chiral Crownpak (CRT) column from J. T. Baker, Inc. ($H_2O$ containing 0.1% by weight $HClO_4$ as the mobile phase, 0.6 ml/min flow rate, 280 nm).

The order of addition of materials to the reaction flask can be such that the acetic acid, the D-ABCS, the first sulfuric acid addition, and the salicylaldehyde are added, followed by acetic anhydride to scavenge the water therefrom, followed by the DL-HPG to initiate the reaction.

Hydrolysis of the D-HPG·D-BCS to Form D-HPG and a Salt of D-BCS, Which Salt can be Recycled

A 250 ml, 3-neck round bottom flask was charged with 10 g. (20.4 mmol) of D-HPG·D-BCS, 1.1 g. (10.4 mmol) of sodium carbonate and 23 g. of methanol. The flask was equipped with a mechanical stirrer, a thermowell, a heating mantel, and a condenser capped with a nirtogen inlet/bubbler system. The flask was under a nitrogen blanket at atmospheric pressure.

The D-HPG·D-BCS formed a slurry in the methanol. The slurry was stirred at about 60°C for a time period ranging from about 6-10 hours, and then cooled to room temperature.

The D-HPG formed was a solid which was filtered from the above-described slurry and washed using about 10 ml of methanol at about 60°C. After during at 40°C under 20 inches of mercury vacuum, 32 g. (94% by weight theoretical yield) of D-HPG having an optical purity of 99.99+ %.

The filtrate containing the sodium salt of bromocamphorsulphonic acid in methanol was added about 3 volumes of acetic acid per volume of methanol. The mixture was then distilled to remove the methanol and the sodium salt of bromocamphorsulfonic acid was recycled to the resolution process without any observed loss of resolution capacity. Ammonium carbonate can be used in this isolation process to produce ammonium salt of bromocamphorsulfonic acid if desired.

FIGS. 1-3 show the HPLC chromatograms for the isomeric mixtures of 3 different compositions. FIG. 1 shows the chromatogram for an initial racemic mixture of DL-HPG of the kind used as the starting material for the above-described resolution-interconversion. The D-HPG is shown at 10 on FIG. 1, with the L-HPG shown at 12. Based on calibration measurements, the FIG. 1 chromatogram shows a 50:50 weight ratio of D-HPG:L-HPG. FIG. 3 shows the chromatogram for the Example 7 product D-HPG. The D-HPG is shown at 30 on FIG. 3, with the L-HPG being undetected. The FIG. 3 chromatogram shows a 99.99+ weight % D-HPG content for the Example 7 product. FIG. 2 is for comparison purposes, as it shows the chromatogram for the highest purity D-HPG presently known to be commercially available. The D-HPG is shown at 20 on FIG. 2, with the L-HPG shown at 22. The FIG. 2 chromatogram shows a 95.5 : 0.5 weight ratio of D-HPG : L-HPG.

Use of an aromatic aldehyde such as salicylaldehyde as the catalyst for the interconversion has been described above. The imine necessary for the interconversion process formed across the $-NH_2$ functionality of the amino acid and the carbonyl functionality of the salicylaldehyde. Thus, one skilled in the art will recognize the possibility of using an aliphatic or aromatic aldehyde or ketone as the catalyst. However, since carbonyl compounds containing an $\alpha$ hydrogen atom undergo self condensation (aldol condensation) under acidic conditions, it is desirable to select a carbonyl compound which is devoid of alpha hydrogens, eg. an aromatic aldehyde.

Examples of aromatic aldehydes which can be used in the present invention include, but are not limited to salicylaldehyde, benzaldehyde, 3,5-dichlorosalicylaldehyde, p-nitrobenzaldehyde, m-nitrobenzaldehyde, and o-nitrobenzaldehyde.

Aliphatic aldehydes devoid of an alpha hydrogen atom can also be used, for example, pivaldehyde.

Independent racemization studies indicated that the rate of interconversion of D-HPG to DL-HPG in acetic

acid is a function of the electronic nature of the aromatic aldehyde. Use of an electron-poor aromatic aldehyde accelerates the rate of racemization. Presence of an electron withdrawing functional group on the aromatic ring results in improved catalytic performance. Table 2 summarizes the results of interconversion studies made using various aromatic aldehydes.

TABLE 2

EFFECT OF AROMATIC ALDEHYDE SELECTION ON INTERCONVERSION OF D-HPG TO DL-HPG

| Example No. | Aldehyde | Temp. (°C) | Time for 100% Interconversion (hours) |
|---|---|---|---|
| 8 | benzaldehyde | 60 | 8 |
| 9 | benzaldehyde | 80 | 3 |
| 10 | salicylaldehyde | 60 | 4 |
| 11 | 3,5-dichlorobenzaldehyde | 60 | 2 |
| 12 | p-nitrobenzaldehyde | 60 | 1 |

Each experiment (Example) was performed in glacial acetic acid using 4 volumes of glacial acetic acid per one volume of D-HPG. The amount of aldehyde used was 10 mol % based on the amount of D-HPG.

Interconversion was measured using chiral HPLC techniques as previously described.

A proper selection of the aldehyde catalyst and the temperature at which resolution-interconversion is carried out can significantly reduce the time required to obtain a high conversion to the desired isomer.

The presence of water (even in trace amounts) has a detrimental effect on the diastereomeric excess obtained during the asymmetric transformation, as previously discussed. Water acts as a solvent for the DL isomeric mixture and thereby prevents complete conversion of the isomeric mixture to D-salt in the resolution-interconversion medium.

Table 3 provides additional data showing the effect of various amounts of water on the diastereomeric excess and conversion obtained in the process previously described for the resolution-interconversion of DL-HPG.

TABLE 3

EFFECT OF WATER ON THE DIASTEREOMERIC EXCESS AND CONVERSION OBTAINED IN THE RESOLUTION-INTERCONVERSION OF DL-HPG TO D-HPG•D-BCS

| Example No. | Amount of Water in Acetic Acid (wt%) | Diastereomeric Excess of Reaction Mixture (%) | Conversion to D-HPG•D-BCS (wt%) |
|---|---|---|---|
| 13 | 0.5 | 90 | 95 |
| 14 | 1.0 | 80 | 90 |
| 15 | 3.0 | 60 | 80 |

The overall reaction conditions and components were the same as those described in Example 7, with the following exceptions: The water content in Example 7 was negligible due to the use of acetic anhydride as a scavenging agent. The Table 3 reaction was carried out at 70°C for a time period of about 18 hours.

Diastereomeric excess was measured by chiral HPLC of a reaction mixture sample quenched in 0.1% aqueous HCl.

When a lower conversion is obtained, there is the possibility of contaminating the desired D-isomer with unwanted DL isomer during the precipitation process.

The highest yields for the process of the present invention were obtained when a calculated amount of acetic anhydride (based on the amount of water present in acetic acid, as measured by Karl-Fisher titration) was added to the acetic acid reaction medium. Since free α-amino acids are also known to react with acetic anhydride, forming the corresponding N-acetyl derivative, the resolution-interconversion was performed wherein acetic anhydride was the last component added to the reaction medium toward the end of the

resolution-interconversion process, thus minimizing the decomposition of DL-HPG.

EXAMPLE 16

Precipitation-Induced Asymmetric Transformation: Stereospecific Synthesis of L-homoalanine-4-yl-(methyl)phosphonic Acid (L-BAST)

The equipment used and reaction conditions are essentially the same as those described for Example 7, except that the equipment is reduced in size to accomodate the reduced quantities of reactant material.

DL-BASTA (ammonium salt),

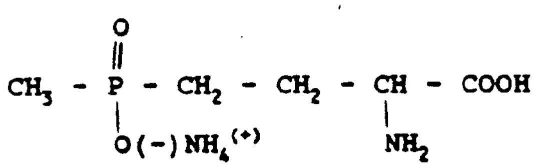

in the amount of 1.66 g (10 mmol) is added to 16 ml of acetic acid; subsequently, 3.28 g (10 mmol) of ammonium 3-bromocamphor-8-sulfonic acid (D-ABCS) is added, followed by 18 ml of 0.5 M sulfuric acid in acetic acid and 50 microliters of salicylaldehyde, to form a reaction slurry.

The above-described slurry is stirred and maintained at a temperature of about 80°C for a time period of about 30 minutes.

A quantity of 2.4 ml of 0.5 M of additional sulfuric acid in acetic acid is then added to the reaction slurry which is at a temperature of about 70°C, over a time period of about 2 hours.

The reaction slurry is then maintained at about 70°C for a time period of about 10 hours, after which the slurry is cooled to about 22°C and filtered to produce 4.4 g of L-homoalanine-4-yl-(methyl)phosphoric acid · D-bromocamphorsulfonic acid (L-BAST·D-BCS), a yield of about 96% by weight based on the DL-BASTA starting material.

The optical purity of this product is 99.9%. L-BAST can then be produced from the L-BAST-D-BCS using the same hydrolysis method as that described in Example 7.

Example 17

Precipitation-Induced Asymmetric Transformation: Stereospecific Synthesis of 3-(3,4-Dihydroxyphenyl)-L-alanine (L-DOPA)

The equipment used and reaction conditions for this Example are essentially the same as those described for Example 7 except that the equipment size is reduced to accommodate the reduced quantities of reactant material.

A mixture of 3-(3,4-dihydroxyphenyl)-DL-alanine (DL-DOPA) (1.97 g, 10 mmol) and acetic acid (16 ml) is contacted with 3.28 g (10 mmol) of ammonium 3-bromocamphor-8-sulfuric acid salt (D-ABCS); subsequently, 9 ml of 0.5M sulfuric acid in acetic acid and 50 microliters of salicylaldehyde are added, to form a reaction slurry.

The above-described slurry is stirred and maintained at a temperatue of about 80°C for a time period of about 30 minutes.

A quantity of 1.2 ml of 0.5M of additional sulfuric acid in acetic acid is then added to the reaction slurry at a temperature of about 70°C over a time period of about 2 hours.

The reaction slurry is then maintained at about 70°C for a time period of about 10 hours, after which the slurry is cooled to about 22°C and filtered to produce 4.96 g of L-DOPA·D-BCS, a yield of about 96% by weight based on the DL-DOPA starting material. Optical purity of the product is about 99.0%. - The L-DOPA·D-BCS can be converted to L-DOPA using the hydrolysis technique described in Example 7.

It will of course be understood that the present invention has been described above purely by way of example and that modifications of detail can be made within the scope of the invention.

The dependencies of the subsidiary claims hereinafter do not imply any limitation as to the possible combinations of the features mentioned in those claims.

## Claims

1. A method of resolving a mixture of α-amino acid stereoisomers or salts thereof, comprising the steps of:
a) contacting an isomeric mixture of an α-amino acid or a salt thereof with at least one chiral resolving agent in an organic acid which is at least a partial solvent for said α-amino acid, in the presence of a mineral acid which is present in a quantity which does not irreversibly protonate the $NH_2$ function of said amino acid, and in the presence of a catalytic amount of an aromatic aldehyde; and,
b) simultaneously carrying out resolution of a desired diastereomer formed by the reaction of said α-amino acid with said chiral resolving agent and diastereomer interconversion of the undesired diastereomer, at a temperature sufficient to promote said resolution-interconversion, at a pressure commensurate with that generated by the resolution-interconversion mixture at said temperature, for a time period sufficient to provide the desired yield of said desired diastereomer at the desired optical purity.

2. The method of Claim 1, wherein said optical resolution is carried out at a temperature less than the boiling point of said organic acid.

3. The method of Claim 2, wherein said temperature ranges between about 60°C and about 120°C.

4. The method of Claim 1, Claim 2, or Claim 3, wherein said resolution-interconversion is done batchwise and the time period of said resolution-interconversion is less than about 24 hours.

5. The method of Claim 1, Claim 2, or Claim 3, wherein said resolution-interconversion is carried out in a continuous process.

6. The method of Claim 1, wherein said α-amino acid or salt thereof is selected from the group consisting of DL-p-hydroxyphenylglycine, DL-homoanaline-4-yl-(methyl)phosphoric acid ammonium salt, and 3-(3,4-dihydroxyphenyl)-DL-alanine.

7. The method of Claim 6, wherein said α-amino acid is DL-p-hydroxyphenylglycine.

8. The method of Claim 7, wherein said chiral resolving agent is (+)-3-bromocamphor-8-sulfonic acid, ammonium salt.

9. The method of Claim 1, wherein said organic acid is selected from the group consisting of $C_1$ to $C_6$ alkanoic acids.

10. The method of Claim 9, wherein said organic acid is acetic acid.

11. The method of Claim 1, wherein said mineral acid is selected from the group consisting of hydrochloric acid, sulfuric acid, phosphoric acid, trifluoromethane sulfonic acid, and toluenesulfonic acid.

12. The method of Claim 11, wherein said mineral acid is a strong mineral acid selected from the group consisting of hydrochloric acid, sulfuric acid, and phosphoric acid, and wherein the concentration of said strong mineral acid during said contacting is less than one equivalent based on the number of equivalents of unreacted α-amino acid or salt thereof.

13. The method of Claim 1, wherein said aromatic aldehyde comprises an electron-withdrawing group on the aromatic ring.

14. The method of Claim 13, wherein said aromatic aldehyde is selected from the group consisting of salicylaldehyde, 3,5-dichlorobenzaldehyde, and m-nitrobenzaldehyde.

15. The method of Claim 14, wherein said aromatic aldehyde is salicylaldehyde.

16. The method of Claim 1, wherein a water scavenging agent is added to said resolution-interconversion mixture to scavange any water present.

17. The method of Claim 16, wherein said water scavenging agent is an anhydride.

18. The method of Claim 17, wherein said anhydride is the anhydride of said organic acid.

**19.** The method of Claim 1, wherein said desired diastereomer obtained thereby is subsequently hydrolyzed to produce the desired optical isomer from said desired diastereomer.

**20.** A method of resolving optically active isomers of an isomeric mixture of DL-p-hydroxyphenylglycine, comprising the steps of:

a) contacting said isomeric mixture of DL-p-hydroxyphenylglycine with (+)-3-bromocamphor-8-sulfonic acid ammonium salt in an organic acid which is at least a partial solvent for said DL-p-hydroxyphenylglycine, in the presence of a mineral acid, which mineral acid is present in a quantity which does not irreversibly protonate the $NH_2$ function of said DL-p-hydroxyphenylglycine, and in the presence of a catalytic amount of an aromatic aldehyde; and,

b) simultaneously carrying out resolution of D-p-hydroxyphenylblycine·(+)-3-bromocamphor-8-sulfonic acid salt while interconverting L-p-hydroxyphenylglycine·(+)-3-bromocamphor-8-sulfonic acid salt to DL-p-hydroxyphenylglycine, over a temperature ranging from about 60°C to about 120°C at a pressure commensurate with that generated by the resolution-interconversion mixture at said temperature, for a time period sufficient to provide the desired yield of said desired diastereomer at the desired optical purity.

**21.** The method of Claim 20, wherein said resolution-interconversion is done batchwise and the time period of said resolution-interconversion is less than about 24 hours.

**22.** The method of Claim 20, wherein said resolution-interconversion is carried out in a continuous process.

**23.** The method of Claim 20, wherein said desired diastereomer obtained thereby is subsequently hydrolyzed to produce the desired optical isomer from said diastereomer.

Fig. 1

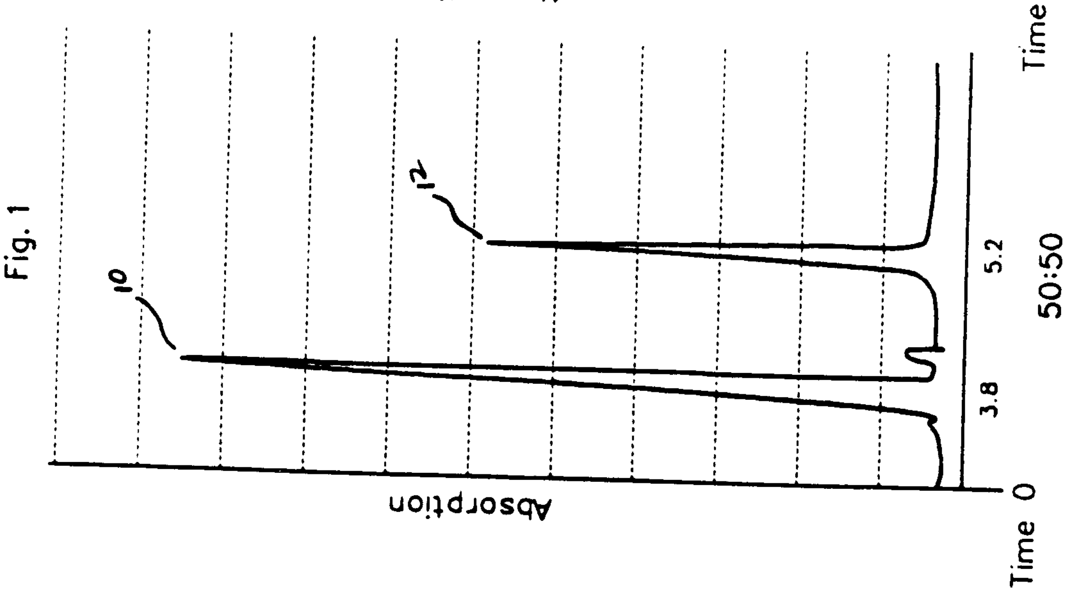


Fig. 2

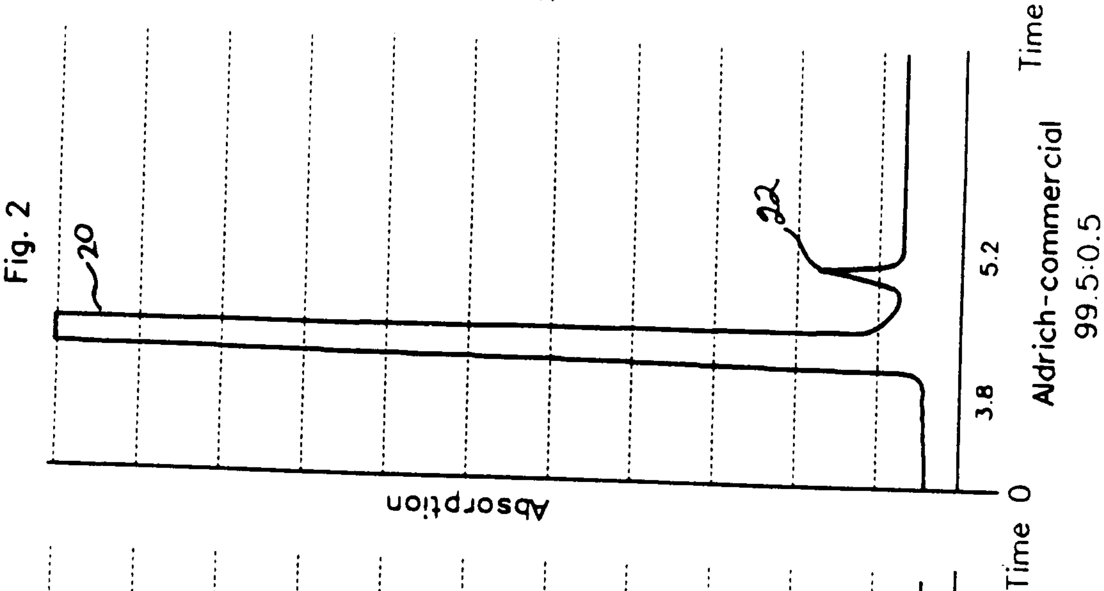


Fig. 3

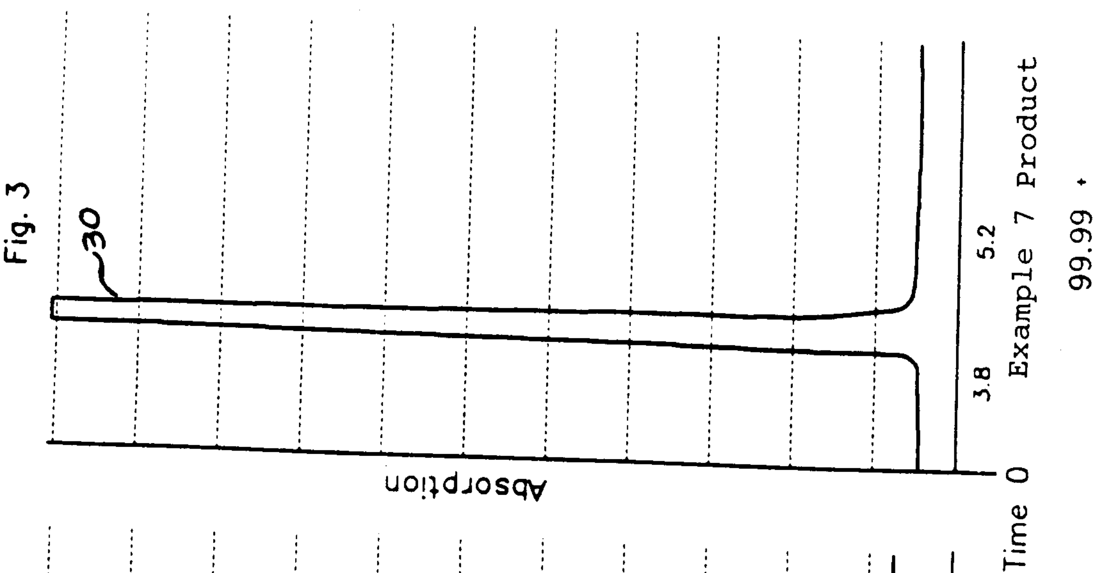

European Patent Office

# PARTIAL EUROPEAN SEARCH REPORT

which under Rule 45 of the European Patent Convention shall be considered, for the purposes of subsequent proceedings, as the European search report

Application Number: EP 92 30 0648

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 173 921 (BEECHAM)<br>* Page 6, lines 19-33; examples; claims * | 1-11,13-15,19-23 | C 07 B 57/00<br>C 07 C 227/30<br>C 07 C 229/36<br>C 07 C 229/34<br>C 07 F 9/30 |
| Y | EP-A-0 057 092 (TANABE SEIYAKU CO. LTD.)<br>* Example 3; claims * | 1-11,13-15,19-23 | |
| A | FR-A-2 438 054 (MEIJI SEIKA KAISHA LTD)<br>* Examples; claims * | 1,6 | |
| D,A | EP-A-0 070 114 (TANABE SEIYAKU CO.LTD)<br>* Examples; claims *<br>-/- | 1-23 | |

TECHNICAL FIELDS SEARCHED (Int. Cl.5)

C 07 B 57/00
C 07 C 227/00
C 07 C 229/00
C 07 F 9/00

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 8,20-23

Claims searched incompletely : 1-7,9-19

Claims not searched :

Reason for the limitation of the search:

The definitions "X amino acid" and "chiral resolving agent" are too broad to allow a complete search. The search has been limited to examples described by the applicant. (Guidelines B III.2.1; 3.12)

| Place of search | Date of completion of the search | Examiner |
|---|---|---|
| THE HAGUE | 07-05-1992 | HELPS I.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

Page 2

European Patent Office

# PARTIAL EUROPEAN SEARCH REPORT

Application Number
EP 92 30 0648

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| D,A | AGRICULTURAL AND BIOLOGICAL CHEMISTRY, vol. 43, February 1979, pages 395-396, Agricultural Chemical Soc. of Japan; S. YAMADA et al.: "Preparation of D-p-hydroxyphenylglycine. Optical resolution of DL-p-hydroxyphenylglycine with d-3-bromocamphor-8-sulfonic acid" * Whole document * --- | 1-23 | |
| D,A | JOURNAL OF ORGANIC CHEMISTRY, vol. 48, no. 6, March 25, 1983, pages 843-846, American Chemical Soc.; S. YAMADA et al.: "Method for the racemization of optically active amino acids" * Whole document * --- | 1-23 | TECHNICAL FIELDS SEARCHED (Int. Cl.5) |
| A | BULLETIN OF THE CHEMICAL SOCIETY OF JAPAN, vol. 58, no. 2, February 1985, pages 433-436, The Chemical Soc. of Japan; C. HONGO et al.: "Asymmetric transformation of DL-p-hydroxyphenylglycine by a combination of preferential crystallization and simultaneous racemization of the o-toluenesulfonate" * Wholedocument * ----- | 1-23 | |

EPO FORM 1503 03.82 (P0410)